(19) **Europäisches Patentamt / European Patent Office / Office européen des brevets**

(11) **EP 4 603 092 A1**

(12) **EUROPEAN PATENT APPLICATION**
published in accordance with Art. 153(4) EPC

(43) Date of publication:
**20.08.2025 Bulletin 2025/34**

(21) Application number: **23877357.6**

(22) Date of filing: **13.10.2023**

(51) International Patent Classification (IPC):
**A61K 31/436** (2006.01)   **A61P 9/00** (2006.01)
**A61P 9/14** (2006.01)   **G16H 50/00** (2018.01)

(52) Cooperative Patent Classification (CPC):
**A61K 31/436; A61P 9/00; A61P 9/14;** G16H 50/00

(86) International application number:
**PCT/JP2023/037150**

(87) International publication number:
**WO 2024/080356 (18.04.2024 Gazette 2024/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.10.2022 JP 2022165311**

(71) Applicants:
• **NATIONAL UNIVERSITY CORPORATION TOKAI
NATIONAL
HIGHER EDUCATION AND RESEARCH SYSTEM
Nagoya-shi, Aichi 464-8601 (JP)**
• **Nobelpharma Co., Ltd.
Chuo-ku
Tokyo 104-0033 (JP)**

(72) Inventors:
• **OZEKI Michio
Gifu-shi, Gifu 501-1193 (JP)**
• **ASADA Ryuta
Gifu-shi, Gifu 501-1193 (JP)**
• **SHIMIZU Kenji
Tokyo 104-0033 (JP)**
• **SANADA Chihiro
Tokyo 104-0033 (JP)**
• **HAMADA Izumi
Tokyo 104-0033 (JP)**

(74) Representative: **Schön, Christoph
Dr. Schön, Neymeyr & Partner mbB
Bavariaring 26
80336 München (DE)**

(54) **THERAPEUTIC AGENT FOR INTRACTABLE LYMPHATIC DISEASE AND INTRACTABLE VASCULAR DISEASE**

(57)   Problems: To provide effective therapeutic agents for the treatment of refractory lymphatic and vascular diseases.

Summary: A therapeutic agent for the treatment of refractory lymphatic and vascular diseases comprising sirolimus as an active ingredient, the administration of which is initiated by oral or tube administration at a dosage determined according to parameters related to the pharmacokinetics of sirolimus in a patient having such diseases and the weight and age of such patient. The therapeutic agent is a therapeutic agent.

EP 4 603 092 A1

FIG. 11

Simulated PK Profiles

**Description**

[Technical Field]

The present invention relates to a drug for improving symptoms of refractory lymphatic and vascular diseases. More specifically, the present invention relates to an oral drug containing sirolimus that is effective in improving symptoms of refractory lymphatic and vascular diseases in children, including infants, and adults, and in treating these diseases.

[Background Art]

Refractory lymphatic and vascular diseases are a group of diseases that mainly occur during childhood, in which abnormalities in the formation of blood vessels and lymphatic vessels occur throughout the body. These diseases often become intractable due to various symptoms such as unilateral hypertrophy and pain in the limbs, ulcers, functional disorders, and organ disorders.

Known examples of refractory lymphatic and vascular diseases include Kaposiform hemangioendothelioma, tufted hemangioma, lymphangioma, lymphangiomatosis, Gorham's disease, venous malformation, blue rubber bleb nevus syndrome, mixed vascular malformation, and Klippel-Trenaunay-Weber syndrome, and are sometimes called intractable vascular tumors, vascular malformations, or intractable vascular abnormalities. Not only can these diseases be fatal if they become severe, but they can also be painful or cause abnormalities in appearance, greatly affecting the patient's quality of life. Therefore, it is extremely important to establish a method of treating these diseases.

There is no established standard treatment for refractory lymphatic and vascular diseases, and no pharmaceuticals that are effective against these diseases. In addition, in current clinical practice, drug therapy using steroids, interferon, propranolol, etc. is sometimes used for cases in which surgery, radiation therapy, etc. are difficult or ineffective, but the effects of the drugs currently used for these diseases are limited, and it is difficult to say that their efficacy and safety have been established. Propranolol is also effective against infantile hemangiomas.

Meanwhile, recent non-clinical studies using sirolimus, a metabolite of the actinomycete Streptomyces hygroscopicus isolated from soil on Easter Island, have suggested that the compound is effective against vascular tumors and vascular malformations (Non-Patent Documents 1 to 5). Clinical studies have also suggested that sirolimus may improve various symptoms of these diseases (Non-Patent Documents 6 and 7).

[Prior art references]

[Non-Patent documents]

Non-Patent document 1: Huber S, Bruns CJ, Schmid G, et al. Inhibition of the mammalian target of rapamycin impedes lymphangiogenesis. Kidney Int 2007; 71:771-777.

Non-Patent document 2: Kobayashi S, Kishimoto T, Kamata S, et al. Rapamycin, a specific inhibitor of the mammalian target of rapamycin, suppresses lymphangiogenesis and lymphatic metastasis. Cancer Sci. 2007 May;98(5):726-33.

Non-Patent document 3: Boscolo E, Coma S, Luks VL, et al. AKT hyper-phosphorylation associated with PI3K mutations in lymphatic endothelial cells from a patient with lymphatic malformation. Angiogenesis. 2015 Apr;18(2):151-62.

Non-Patent document 4: Boscolo E, Limaye N, Huang L, et al. Rapamycin improves TIE2-mutated venous malformation in murine model and human subjects. J Clin Invest. 2015; 125: 3491-604.

Non-Patent document 5:Limaye N, Kangas J, Mendola A, et al. Somatic Activating PIK3CA Mutations Cause Venous Malformation. Am J Hum Genet. 2015 Dec 3;97(6):914-21.

Non-Patent document 6: Adrienne M. Hammill, et al. Sirolimus for the Treatment of Complicated Vascular Anomalies in Children, Pediatr Blood Cancer, 2011, 57, 1018-24.

Non-Patent document 7: Ozeki M, Nozawa A, Yasue S, Endo S, Asada R, Hashimoto H, Fukao T. The impact of sirolimus therapy on lesion size, clinical symptoms, and quality of life of patients with lymphatic anomalies. Orphanet J Rare Dis. 2019 Jun 13;14(1):141.

[Summary of Invention]

[Problems to be resolved by the invention]

For refractory lymphatic and vascular diseases (hereinafter referred to as "these diseases"), there is no known therapeutic agent with fully proven efficacy. At present, sirolimus is the most promising candidate drug, but when used to treat these diseases, it is desirable to use an effective and safe method of administration and dosage. This is particularly important when used in children, including infants. The present application has been made in view of the above circumstances, and aims to provide a drug that is safe and effective for the treatment of refractory lymphatic and vascular diseases.

[Means of solving the problems]

The present invention provides the following embodiments.

Embodiment 1:

A therapeutic agent, comprising sirolimus as an active ingredient, for the treatment of refractory lymphatic and vascular diseases administered by oral or tube administration with an initial dose of the dosage determined by parameters related to the pharmacokinetics of sirolimus, and the patient's body weight and age in months.

Embodiment 2:

The therapeutic agent according to embodiment 1, wherein the initial dose is calculable by:

using patient information including at least age in months and body weight, and dosing information including at least the dosage and timing of administration of the drug, calculating parameters corresponding to patient information using a parameter calculation formula for calculating parameters to be applied to a blood concentration calculation formula representing the blood sirolimus concentration based on a two-compartment model upon repeated administration, and
applying the parameters and the administration information to the blood concentration calculation formula to obtain a curve showing the change in blood sirolimus concentration, and applying a target value of the blood sirolimus concentration to the curve showing the change in blood sirolimus concentration,
wherein the patient's age in months used to determine the parameters at each time point is the patient's age at each time point calculated based on the date of birth, and the patient's weight used to determine the parameters at each time point is the patient's estimated weight at each time point estimated based on the patient's past weight change trends.

Embodiment 3:

A therapeutic agent, comprising sirolimus as an active ingredient, for the treatment of refractory lymphatic and vascular diseases administered by oral or tube administration with an initial dose shown below depending on the weight and age in months of the patient with the disease or condition.

(1) Patients weighing 30 kg or more: 1.4 mg/day of sirolimus
(2) Patients weighing less than 30 kg:

Less than 3 months old 0.02 mg/kg/day as sirolimus
3 months to less than 6 months of age 0.04 mg/kg/day of sirolimus
6 months to less than 12 months of age 0.06 mg/kg/day of sirolimus
12 months and older 0.08 mg/kg/day of sirolimus
The maximum initial dose should be 1.4 mg/day of sirolimus per patient

Embodiment 4:

The therapeutic agent according to any one of embodiments 1 to 3, wherein the dosage is adjusted based on the measured blood sirolimus concentration after repeated administration of the same dosage for a certain period of time, so that the blood trough level of sirolimus in the patient is adjusted to be 5 ng/mL or more and 15 ng/mL or less.

Embodiment 5:

The therapeutic agent according to any one of embodiments 1 to 3, wherein the dosage is increased or decreased based on clinical symptoms after repeated administration at the same dosage for a certain period of time.

Embodiment 6:

The therapeutic agent according to any one of embodiments 1 to 5, wherein the refractory lymphatic and vascular diseases is selected from the group consisting of Kaposi type hemangioendothelioma, tufted hemangioma, lymphangioma, lymphangiomatosis, Gorham disease, venous malformation, blue rubber ball-like nevus syndrome, mixed type vascular malformation, and Klippel-Trenaunay-Weber syndrome.

Embodiment 7:

The therapeutic agent according to any one of embodiments 1-6, wherein the therapeutic agent is a granule.

Embodiment 8:

A method for treating refractory lymphatic and vascular diseases, comprising: (a) determining an initial dose of sirolimus based on parameters related to the pharmacokinetics of sirolimus, as well as the patient's weight and age; and (b) administering a pharmaceutical composition containing sirolimus by oral or tube administration.

Embodiment 9:

A method for treating refractory lymphatic and vascular diseases, comprising administering by oral or tube administration a pharmaceutical composition containing sirolimus with an initial dose shown below.

(1) Patients weighing 30 kg or more: 1.4 mg/day of sirolimus
(2) Patients weighing less than 30 kg:

Less than 3 months old 0.02 mg/kg/day as sirolimus
3 months to less than 6 months of age 0.04 mg/kg/day of sirolimus
6 months to less than 12 months of age 0.06 mg/kg/day of sirolimus
12 months and older 0.08 mg/kg/day of sirolimus
The maximum initial dose should be 1.4 mg/day of sirolimus per patient

Embodiment 10:

A use of sirolimus for the preparation of a medicine for treating refractory lymphatic and vascular diseases, wherein the initial dose of sirolimus is determined according to parameters related to the pharmacokinetics of sirolimus, as well as the body weight and age of the patient, and sirolimus is administered by oral or tube administration.

[Effects of the Invention]

The present invention makes it possible to administer an effective and minimum dose of sirolimus for treating refractory lymphatic and vascular diseases or improving symptoms, and as a result, it becomes possible to treat refractory lymphatic and vascular diseases or improve symptoms safely while reducing the burden on patients (particularly children including infants) and achieving QOL.

[Mode for carrying out the invention]

The therapeutic agent for refractory lymphatic and vascular diseases according to the present invention will be specifically described below.
In the present invention, refractory lymphatic disease is a general term for refractory diseases caused by abnormalities in lymphatic vessels, including lymphangioma (lymphatic vascular malformation), lymphangiomatosis, Gorham's disease, and lymphangiectasia.
In the present invention, refractory vascular disease is a general term for refractory diseases caused by abnormalities in blood vessels, including hemangioendothelioma, tufted hemangioma, venous malformation, blue rubber bleb nevus syndrome, mixed vascular malformation, and Klippel-Trenaunay-Weber syndrome.
Although refractory lymphatic diseases and refractory vascular diseases are distinct in terms of disease name, they are both diseases caused by abnormal formation of blood vessels or lymphatic vessels, and have been published as one treatment guideline (Guidelines for the Treatment of Hemangiomas, Vascular Malformations, and Lymphatic Malformations 2017). Therefore, in the present invention, these diseases are collectively referred to as "refractory lymphatic and vascular diseases" and are the subject of treatment.

In the present invention, the refractory lymphatic and vascular diseases to be treated include mixed lymphatic and vascular diseases, which can also be referred to as vascular tumors and vascular malformations or vascular abnormalities.

The therapeutic agent of the present invention is a therapeutic agent for one or more diseases or symptoms selected from refractory lymphatic vascular diseases, refractory vascular diseases, and symptoms associated with these diseases, containing sirolimus as an active ingredient, and is administered by oral or tube administration, with an initial dose determined according to parameters related to the pharmacokinetics of sirolimus, as well as the body weight and age of the patient.

In detail, the therapeutic agent of the present invention is a therapeutic agent for refractory lymphatic and vascular diseases, containing sirolimus as an active ingredient, and is characterized in that the administration is started by oral or tube administration at a dosage determined according to parameters related to the pharmacokinetics of sirolimus (blood hemoglobin concentration, dosage form (tablets or granules), storage state of the sample for blood concentration determination (frozen or refrigerated storage), presence or absence of concomitant drug (particularly CYP3A4 inducers or inhibitors)) and the weight and age of the patient.

In a preferred embodiment of the present invention, the initial dosage can be calculated based on a theoretical formula that represents the pharmacokinetics in the patient's body after administration of sirolimus. Specifically, the dosage and actual measured values of the blood sirolimus concentration in a patient having a target disease are applied to a theoretical formula such as a two-compartment model to calculate each unique parameter, and the dosage suitable for the patient can be calculated based on the theoretical formula completed using the parameters. More specifically, the dosage suitable for the patient can be calculated from the following method for estimating the blood sirolimus concentration.

Method for estimating blood sirolimus concentration

In a preferred embodiment, the initial dose of sirolimus in the present invention can be determined using a blood sirolimus concentration estimation system that estimates the blood sirolimus concentration.

In a preferred embodiment, the blood sirolimus concentration estimation system used for estimating the blood sirolimus concentration in the present invention is configured as follows.

That is, the system estimates the change in blood sirolimus concentration in a patient who has been repeatedly administered a drug containing sirolimus as an active ingredient, and includes a patient information input unit which accepts input of patient information including at least age in months and body weight, an administration information input unit which accepts input of administration information including at least the dose and administration timing of the drug, a memory unit which stores a blood concentration calculation formula expressing the blood sirolimus concentration based on a two-compartment model upon repeated administration and a parameter calculation formula for calculating parameters to be applied to the blood concentration calculation formula, a parameter calculation unit which calculates parameters corresponding to the patient information using the parameter calculation formula read from the memory unit, and an estimated curve calculation unit which applies the parameters and the administration information to the blood concentration calculation formula to obtain a curve expressing the change in blood sirolimus concentration, wherein the patient's age in months used to determine the parameters at each time point is the patient's age at each time point calculated based on the date of birth, and the patient's weight used to determine the parameters at each time point is the patient's estimated weight at each time point estimated based on the change in weight change in the patient's past.

With this configuration, the age and weight in months at the time point at which the blood sirolimus concentration is estimated are applied to the parameter calculation formula to calculate the parameters, and the blood sirolimus concentration can be appropriately determined. In particular, since the weight of infants changes greatly with age and it is difficult to predict the clearance, estimating the change in blood sirolimus concentration makes it possible to create an appropriate dosing plan.

The blood sirolimus concentration estimation system may further include a dosage form information input unit that accepts input of information on the dosage form of the drug, and the parameter calculation unit may determine parameters based on the information on the dosage form. With this configuration, the parameters of the blood concentration calculation formula can be determined considering the dosage form of the sirolimus preparation as a variable factor, and the blood sirolimus concentration transition can be estimated. The parameter calculation unit may also determine parameters based on drug concentration assay information.

The blood sirolimus concentration estimation system may further include a concomitant drug information input unit that accepts input of information on concomitant drugs, and the parameter calculation unit may determine parameters based on the information on the concomitant drugs. With this configuration, the parameters of the blood concentration calculation formula can be determined considering the concomitant drugs as a variable factor, and the blood sirolimus concentration transition can be estimated. Here, the concomitant drug to be input in the concomitant drug information input unit may be a CYP3A4 inhibitor or a CYP3A4 inducer, or may be another drug that affects the clearance of sirolimus, or a complication such as liver damage, etc.

The blood sirolimus concentration estimation system includes an actual measured value input unit that accepts input of an actual measured value of the blood concentration of the drug at least one arbitrary time point in the patient, and a parameter correction unit that estimates the parameters ex post by Bayesian estimation using the actual measured value, and the estimated curve calculation unit may obtain a curve that represents the blood concentration transition using the ex post estimated parameters. With this configuration, the blood sirolimus concentration transition specific to the patient can be estimated.

The blood sirolimus concentration estimation system may further include a recommended dose calculation unit that calculates a recommended dose of the drug by applying a target value of the blood sirolimus concentration to a curve representing the blood concentration transition. With this configuration, a recommended dose for controlling the blood sirolimus concentration change to the target value is calculated. The target value may be set by a trough value.

In a preferred embodiment, the method for estimating blood sirolimus concentration used in the present invention is a method for estimating a change in blood sirolimus concentration in a patient who has been repeatedly administered a drug containing sirolimus as an active ingredient, and comprises the steps of: accepting input of patient information including at least age in months and body weight; accepting input of administration information including at least the dosage and administration timing of the drug; reading from a memory unit a blood concentration calculation formula expressing the blood sirolimus concentration based on a two-compartment model upon repeated administration and a parameter calculation formula for calculating parameters to be applied to the blood concentration calculation formula; calculating parameters corresponding to the patient information using the parameter calculation formula read from the memory unit; and applying the parameters and the administration information to the blood concentration calculation formula to obtain a curve expressing a change in blood sirolimus concentration; the patient's age in months used to determine the parameters at each time point is the patient's age in months at each time point calculated based on the date of birth, and the patient's weight used to determine the parameters at each time point is the patient's estimated weight at each time point estimated based on the change in past body weight change. The method for estimating the blood sirolimus concentration may include various configurations of the above-mentioned system for estimating the blood sirolimus concentration. For example, the parameters at each time point may be determined using information on the concomitant use of a CYP3A4 inhibitor or a CYP3A4 inducer, dosage form information of the sirolimus formulation, and drug concentration assay information.

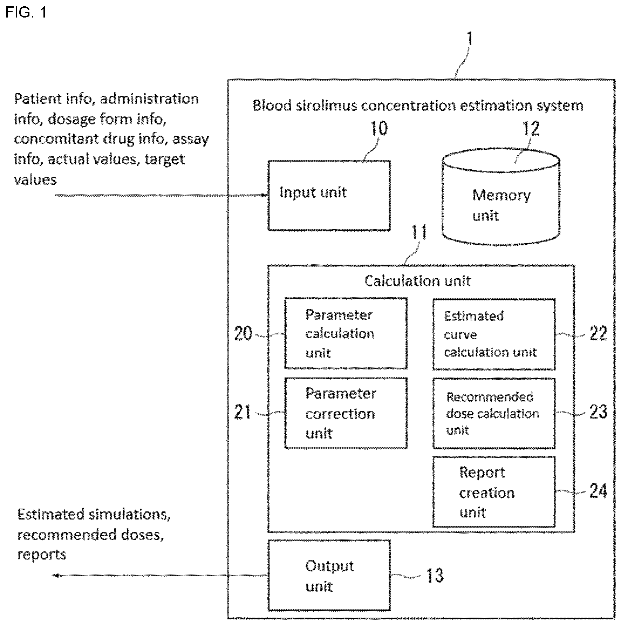

The configuration and operation of the blood sirolimus concentration estimation system used to determine the initial dosage in the present invention will be described below. FIG. 1 is a diagram showing the configuration of a blood sirolimus concentration estimation system 1 in a preferred embodiment. Blood sirolimus concentration estimation system 1 has an input unit 10 that accepts input of various data, a calculation unit 11 that estimates the blood sirolimus concentration trend based on the input data, a memory unit 12 that stores data used in the calculation, and an output unit 13 that outputs an estimated curve of the estimated blood sirolimus concentration trend and a recommended dose of the drug.

Input unit 10 accepts input of patient information including the patient's date of birth and weight, administration information regarding the dosage and administration timing of the drug, information on the drug's dosage form (tablet/granule/pulverized), and information on concomitant drug. In this form, information on a CYP3A4 inhibitor or CYP3A4 inducer is accepted as the concomitant drug. Here, the concomitant drug information may be the rate of change of a pharmacokinetic parameter (particularly, but not limited to, CL/F) when the concomitant drug is taken together with the drug. The input unit 10 also accepts data on the actual measured value of the patient's blood sirolimus concentration and data on the target value.

The memory unit 12 stores pre-constructed PK (pharmacokinetics) model information. Specifically, it stores a blood concentration calculation formula for the active ingredient sirolimus when the drug is repeatedly administered, a parameter calculation formula for calculating parameters corresponding to patient information, and pre-constructed estimated values of pharmacokinetic parameters. The blood concentration calculation formula is a formula that expresses the blood sirolimus concentration based on a two-compartment model. The parameters corresponding to patient information are parameters used in the blood concentration calculation formula, and are standard parameters for estimating the blood sirolimus concentration of patients who have the same patient information (also referred to as "patient background"). In this document, these are referred to as "standard PK parameters".

In an embodiment of the blood sirolimus concentration estimation system, the blood concentration calculation formula is represented by the following formula (1).

[Formula 1]

$$C(t) = \sum_{i=1}^{n} D_i \left\{ A e^{-\alpha\left(t-t_{D_i}\right)} + B e^{-\beta\left(t-t_{D_i}\right)} - (A+B)e^{-k_a\left(t-t_{D_i}\right)} \right\} \quad \cdots (1)$$

$$A = \frac{k_a}{V_1/F} \frac{\frac{Q}{V_2/F} - \alpha}{(k_a-\alpha)(\beta-\alpha)} \qquad B = \frac{k_a}{V_1/F} \frac{\frac{Q}{V_2/F} - \beta}{(k_a-\beta)(\alpha-\beta)}$$

$$\alpha = \frac{\frac{Q/F \; Cl/F}{V_2/F V_1/F}}{\beta} \qquad \beta = \frac{1}{2}\left[ \frac{Q/F}{V_1/F} + \frac{Q/F}{V_2/F} + \frac{Cl/F}{V_1/F} - \sqrt{\left(\frac{Q/F}{V_1/F} + \frac{Q/F}{V_2/F} + \frac{Cl/F}{V_1/F}\right)^2 - 4\frac{Q/F}{V_2/F}\frac{Cl/F}{V_1/F}} \right]$$

Here, ka is the absorption rate constant (h-1), Cl/F is the clearance (L/h), $V_1/F$ is the volume of the first compartment (L), $V_2/F$ is the volume of the second compartment (L), and Q/F is the intercompartmental clearance (L). The values of these parameters are related to the patient's weight and age, the dosage form of the administered drug, and the presence or absence of concomitant use of a CYP3A4 inhibitor or CYP3A4 inducer. $D_i$ is the dose of the drug.

Each parameter in the blood concentration calculation formula is expressed as a function with the patient's weight and age, the dosage form of the drug, etc. as variables. This function corresponds to the parameter calculation formula for calculating the parameters corresponding to the patient. This parameter calculation formula is obtained in advance by population pharmacokinetic analysis using a nonlinear mixed effects model.

The calculation formula for calculating the parameters corresponding to the patient will be specifically explained. In one example, the calculation formula for the absorption rate constant ka is expressed by the following formula (2).
[Formula 2]

$$k_a = TVk_a \times [\,4.26 \text{ if Granule formulation }] \quad \cdots (2)$$

In formula (2), TVka is the typical value of the absorption rate constant ka, and is obtained in advance by population pharmacokinetic analysis. In this embodiment, the point estimate of TVKa is 0.235 (h-1), the relative error is 1.17%, and the 95% confidence interval is 0.229-0.240. In addition, "Granule formulation" indicates granules, and formula (2) indicates that when the drug is a granule, TVka is multiplied by 4.26.

The calculation formula for clearance Cl/F is expressed by the following formula (3).
[Formula 3]

$$Cl/F = TVCl \times \left(\frac{WT}{70}\right)^{0.75} \times \frac{1}{1+e^{\left(-2.94\cdot\log\left(\frac{PMA}{62.9}\right)\right)}} \times [\,1.92 \text{ if Clinical trials assay }] \times$$
$$[CYP3A4 \, fold \, change] \times e^{\eta CL} \quad \cdots (3)$$

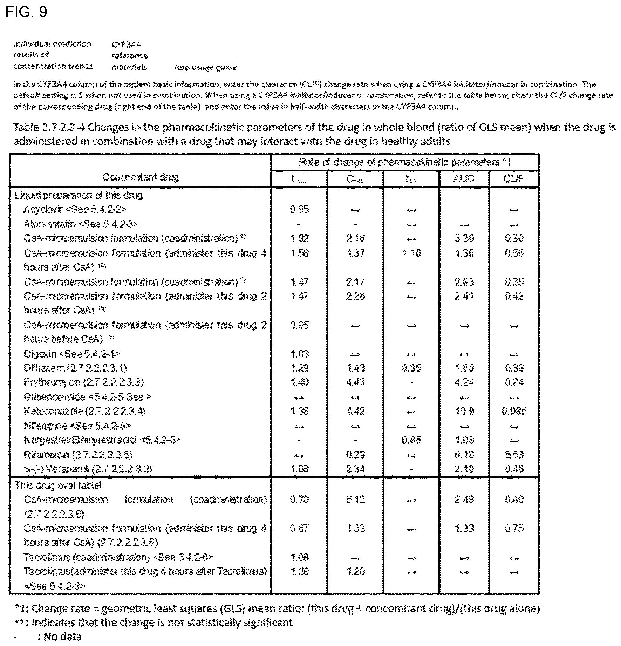

In formula (3), TVCl is a typical value of clearance Cl/F, and is obtained in advance by population pharmacokinetic analysis. In this embodiment, the point estimate of TVCl is 6.44 (L/h), the relative error is 6.04%, and the width of the 95% confidence interval is 5.72-7.25. WT is the patient's body weight. PMA (Post menstrual age) is the age after the last menstrual period (gestational age + postnatal age) and corresponds to the patient's age. $e^{\eta CL}$ represents inter-individual variation. When there is no actual measured value for the patient, $\eta$CL is 0, and the inter-individual variation is 1. "Clinical trials assay" refers to a blood sample stored frozen, and in the case of a blood sample stored frozen, it is multiplied by 1.92. In the case of a blood sample stored refrigerated, it is multiplied by 1. "CYP3A4 fold change" is a coefficient that is determined according to the concomitant drug when there is a concomitant drug. The coefficient will be described later with reference to FIG. 9.

The calculation formula for the volume of the first compartment, $V_1/F$, is expressed by the following formula (4).
[Formula 4]

$$V_1/F = TVV_1 \times \left(\frac{WT}{70}\right)^{1} \times e^{\eta V_1} \quad \cdots (4)$$

In formula (4), $TVV_1$ is a typical value of the volume of the first compartment $V_1/F$, and is obtained in advance by population pharmacokinetic analysis. In the embodiment, the point estimate of $TVV_1$ is 176 (L), the relative error is 2.43%, and the width of the 95% confidence interval is 168-185. WT is the patient's body weight. $e^{\eta V_1}$ represents inter-individual variation. When there is no actual measured value for the patient, $\eta V_1$ is 0, and the inter-individual variation is 1. Depending on the information that can be obtained, it may be difficult to estimate the random effect of the patient on $V_1$, and in such a case, the inter-individual variation for $V_1$ may be set to 1 for all patients.

The calculation formula for the volume of the second compartment, $V_2/F$, is given by the following formula (5).

[Formula 5]

$$V_2/F = TVV_2 \times \left(\frac{WT}{70}\right)^1 \qquad \cdots \quad (5)$$

In formula (5), $TVV_2$ is the typical value of the volume of the second compartment $V_2/F$, and is determined in advance by population pharmacokinetic analysis. In this embodiment, the point estimate of $TVV_2$ is 281 (L), the relative error is 13%, and the width of the 95% confidence interval is 218-362. WT is the patient's body weight.

The calculation formula for the intercompartmental clearance Q/F is expressed by the following formula (6).

[Formula 6]

$$Q/F = TVQ \times \left(\frac{WT}{70}\right)^{0.75} \qquad \cdots \quad (6)$$

In this formula, TVQ is the typical value of the intercompartmental clearance Q/F, which is obtained in advance by population pharmacokinetic analysis. In this embodiment, the point estimate of TVQ is 33.6 (L), the relative error is 4.07%, and the width of the 95% confidence interval is 31.0-36.3. WT is the patient's body weight.

The calculation unit 11 has a parameter calculation unit 20, a parameter correction unit 21, an estimated curve calculation unit 22, a recommended dose calculation unit 23, and a report creation unit 24. These functions are realized by the calculation unit 11 executing a program for estimating the blood sirolimus concentration.

The parameter calculation unit 20 has a function of calculating standard PK parameters (absorption rate constant ka, clearance Cl/F, volume of the first compartment $V_1/F$, volume of the second compartment $V_2/F$, intercompartmental clearance Q/F) corresponding to the patient information. The parameter calculation unit 20 calculates standard PK parameters corresponding to the patient information, using the data of the patient's date of birth and weight input from the input unit 10. The details of the process will be described later.

The parameter correction unit 21 has a function of updating the standard PK parameters after the fact using the actual measured value of the sirolimus concentration in the patient's blood. The standard PK parameters (ka, Cl/F, $V_1/F$, $V_2/F$, Q/F) have a stochastic variance. The variance is reduced using the patient's actual measured value to obtain the PK parameters specific to that patient. In this document, these are referred to as "patient PK parameters." As an example, the clearance Cl/F will be explained. The TVCI of the final model of Cl/F and its variance are set as the conjugate prior distribution, and the patient's actual measured value and its variance are set as the likelihood, and the conjugate posterior distribution is obtained according to Bayes' theorem. Specifically, the conjugate posterior distribution is obtained by multiplying the conjugate prior distribution by the likelihood. The median TVCI of the updated distribution obtained in this way becomes the TVCI specific to that patient.

The estimated curve calculation unit 22 has a function of applying the standard PK parameters calculated by the parameter calculation unit 20 or the patient PK parameters obtained by correcting them by the parameter correction unit 21 and the administration information Di to the blood concentration calculation formula (formula (1)) to obtain a curve showing the blood concentration transition of sirolimus with drug administration. The curve showing the blood concentration transition obtained using the standard PK parameters is a standard concentration transition curve for a patient of the same age and weight, while the curve showing the blood concentration transition obtained using the patient PK parameters is a patient-specific blood concentration transition curve reflecting the data of that patient.

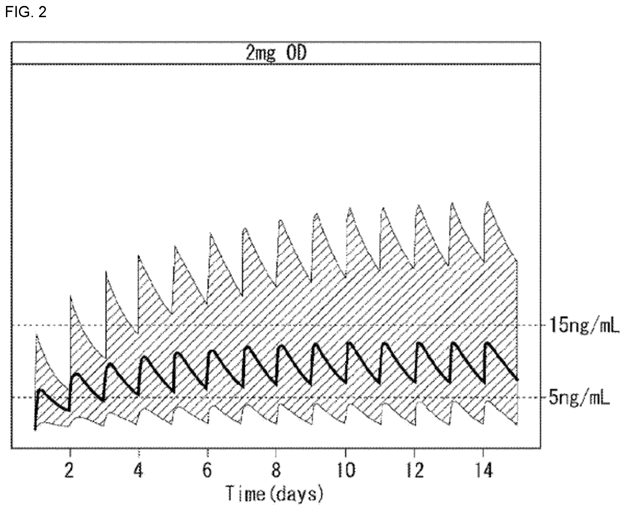

FIG. 2 shows a standard transition curve of blood sirolimus concentration when a patient aged 7 years or older (body surface area 1.0 to less than 1.5 m$^2$) is administered a 2 mg tablet once a day. As shown in gray in FIG. 2, the transition of blood sirolimus concentration is estimated with a 90% prediction interval. In other words, there is a 90% probability that the blood sirolimus concentration of patients of the same age and weight will fall within the range shown in gray. In FIG. 2, the bold curve shows the progress of the blood sirolimus concentration calculated using the point estimate of the Typical Value as the standard PK parameter.

The recommended dose calculation unit 23 calculates the recommended dose of the drug containing sirolimus as an active ingredient by applying the target value of the blood sirolimus concentration to a curve representing the blood concentration transition. The report creation unit 24 has a function of creating a report on the blood sirolimus concentration transition. The output unit 13 has a function of outputting the calculation result of the blood sirolimus concentration transition, the recommended dose of the drug, and a report.

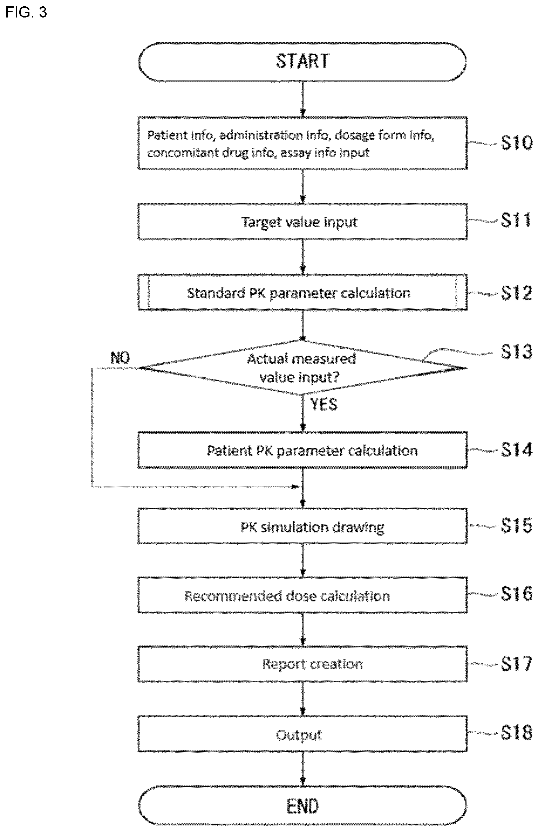

FIG. 3 is a flowchart showing the operation of the blood sirolimus concentration estimation system 1. The blood sirolimus concentration estimation system 1 accepts input of patient information, administration information, dosage form information, concomitant drug information of a CYP3A4 inhibitor or CYP3A4 inducer, and drug concentration assay information (S10). The patient information is data on the patient's date of birth and weight, and the administration information is data on the drug dosage (e.g., 1 mg, 2 mg, etc.) and administration timing (once a day, twice a day, etc.). The dosage form information indicates whether the drug administered to the patient is a granule or a tablet. The concomitant drug information of the CYP3A4 inhibitor or CYP3A4 inducer may be the rate of change of the pharmacokinetic parameters when the concomitant drug is taken together with the present drug. This rate of change will be described later with reference to FIG. 9. The drug concentration assay information indicates whether the blood sample was frozen or refrigerated. In addition, if a parameter that affects the blood concentration calculation formula (e.g., hemoglobin amount, etc.) becomes clear in the future, the new parameter may be input as patient information.

The blood sirolimus concentration estimation system 1 also accepts input of a target value of the blood sirolimus concentration (S11). In this embodiment, the target value is the trough value of the blood sirolimus concentration. Next, the blood sirolimus concentration estimation system 1 calculates the standard PK parameters based on the input patient information, administration information, and dosage form information (S12).

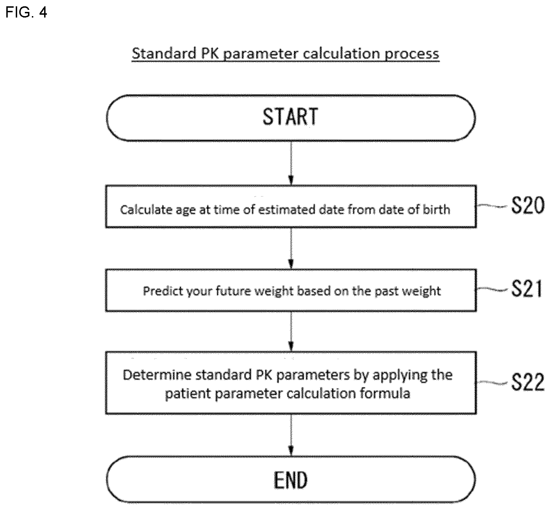

FIG. 4 is a flow chart showing the calculation process of the standard PK parameters. The blood sirolimus concentration estimation system 1 calculates the age (months) at the time of estimating the blood concentration from the patient's date of birth (S20). In addition, the system predicts the weight at the time of estimating the blood concentration from the patient's weight data (S21). In order to predict the weight, it is preferable that the weight data input as patient information is data showing the progress of weight changes. In other words, it is preferable that there is weight data at a plurality of time points. For example, the weight data at three time points is linearly regressed to predict the patient's weight for the next three months. As an example, three months (90 days) are divided into 500 parts, and the weight at each time point is predicted. The blood sirolimus concentration estimation system 1 applies the age at the time of estimation and the predicted weight data to the parameter calculation formula to determine the standard PK parameters to be used at each estimation time point (S22). The blood sirolimus concentration estimation system 1 may perform linear regression of future weight from the input weight data as described above to obtain the weight predicted at the estimation time point, or may use the final weight data after the final weight data. Whether to perform linear regression or use the final weight data may be determined according to the number of weight data input. If the weight can be estimated accurately by linear regression, linear regression may be performed, and if linear regression does not work, the final weight data may be used. For example, linear regression may be performed when the number of input data points is three or more, and the final weight data may be used when the number of input data points is less than three, but this threshold is not limited to three points and may be two points or four points or more.

Referring back to FIG. 3, the blood sirolimus concentration estimation system 1 determines whether or not the actual measured value of the patient's blood sirolimus concentration has been input (S13). If the actual measured value of the patient has been input (YES in S13), the actual measured value is used to update the standard PK parameters and calculate the patient PK parameters (S14). If the actual measured value has not been input (NO in S13), the standard PK parameters are used as is.

The blood sirolimus concentration estimation system 1 generates a blood concentration calculation formula using standard PK parameters or patient PK parameters at the estimation time point, and applies administration information to the blood concentration calculation formula to estimate the blood sirolimus concentration at each estimation time point and perform a PK simulation (S15). The blood sirolimus concentration estimation system 1 applies a target value to the obtained blood concentration trend to calculate the recommended dose of the drug (S16). Next, the blood sirolimus concentration estimation system 1 creates a report summarizing the blood sirolimus concentration trend, the recommended dose, etc. (S17), and outputs the created report (S18).

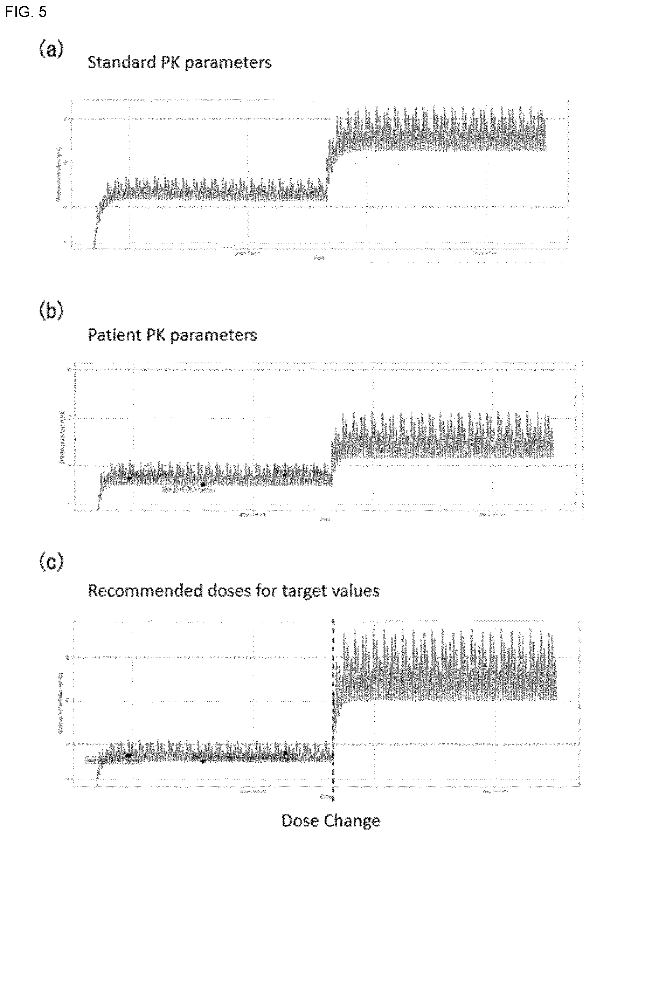

FIG. 5(a) shows the blood sirolimus concentration transition estimated using standard PK parameters applied with patient information, and FIG. 5(b) shows the blood sirolimus concentration transition estimated by updating the standard PK parameters using the actual measured values of the patient's blood sirolimus concentration (three points on February 13, March 13, and April 13, 2021) to obtain patient-specific PK parameters. As can be seen by comparing FIG. 5(a) and FIG. 5(b), the blood sirolimus concentration corrected by the actual measured values transitions lower than the standard blood sirolimus concentration, resulting in a patient-specific concentration transition that includes the actual measured values.

FIG. 5(c) shows the blood sirolimus concentration transition when a drug corresponding to the target value is administered when 10 ng/mL is input as the target trough value. As can be seen by comparing FIG. 5(b) and FIG. 5(c), by changing to the recommended dose corresponding to the target value, the trough value was increased to 10 ng/mL.

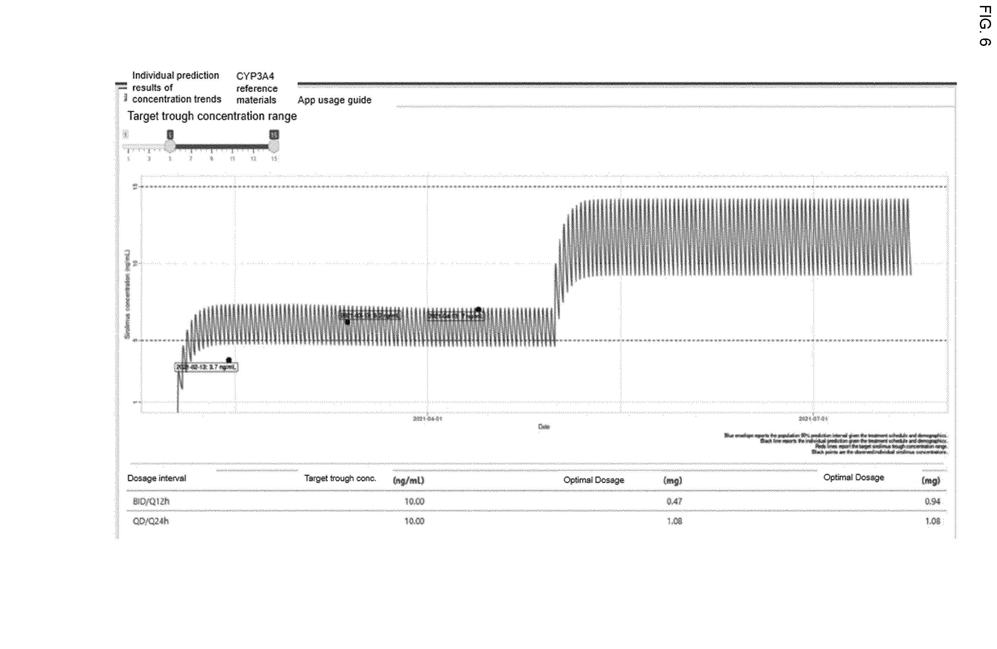

FIGs. 6 to 9 are diagrams showing examples of output screens of the blood sirolimus concentration estimation system 1 of this embodiment. These output screens are screens created by the report creation unit 24. As shown in FIG. 6, the output screen has tabs at the top of the screen for "Individual Prediction Results of Concentration Trends," "CYP3A4 Reference Materials," and "Guide to Using the App."

FIG. 6 shows an example of a screen when the tab for "Individual Prediction Results of Concentration Trends" is selected. This tab displays a graph of the individual prediction results of concentration trends predicted from the patient information and administration information of the selected patient and the actual measured values. The vertical axis of the graph is the blood sirolimus concentration, and the horizontal axis is the time axis. The black circles in the graphs indicate the actual measured values of the trough values of the blood sirolimus concentration of the patient.

Between the tabs at the top of the screen and the graph, there is a slider for setting the "target trough concentration range." The round buttons on this slider allow the user to set the upper and lower limits of the target trough concentration range. The set upper and lower limits are displayed as dotted lines extending horizontally on the graph. In the example shown in FIG. 6, the upper limit is 15 ng/mL and the lower limit is 5 ng/mL. By displaying the upper and lower limits on the graph in this way, the target trough concentration range can be grasped at a glance.

At the bottom of the screen shown in FIG. 6, information on the optimal drug dosage based on the simulation results is displayed. "Dosage interval," "target trough concentration (ng/mL)," "optimum single dose (mg)," and "optimum daily dose (mg)" are listed. These are the dosages that were the premise of the concentration prediction mentioned above. In the example shown in FIG. 6, the optimal dose per dose to achieve a target trough concentration of 10 ng/mL at a dosing interval of BID/Q12h (twice a day) is 0.47 mg, and the optimal daily dose is 0.94 mg. The optimal dose per dose to achieve a target trough concentration of 10 ng/mL at a dosing interval of QD/Q24h (once a day) is 1.08 mg, and the optimal daily dose is 1.08 mg.

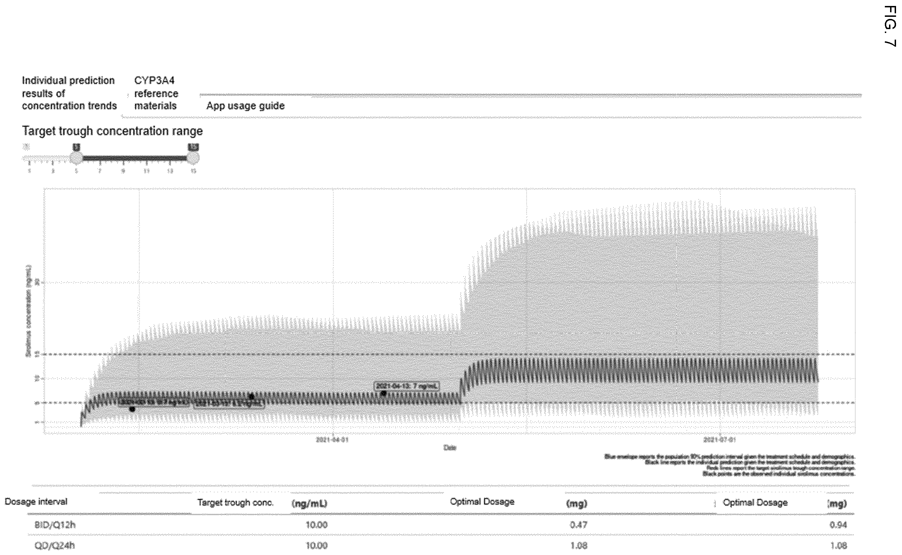

FIG. 7 shows another example of the screen that appears when the tab "Individual prediction results of concentration trends" is selected. The example screen shown in FIG. 7 displays the 90% prediction interval of blood sirolimus concentrations for patients with the same patient background, such as age, weight, and concomitant drug information. This allows you to check how much the concentration trends of individual patients deviate from the average value for a population with the same patient background.

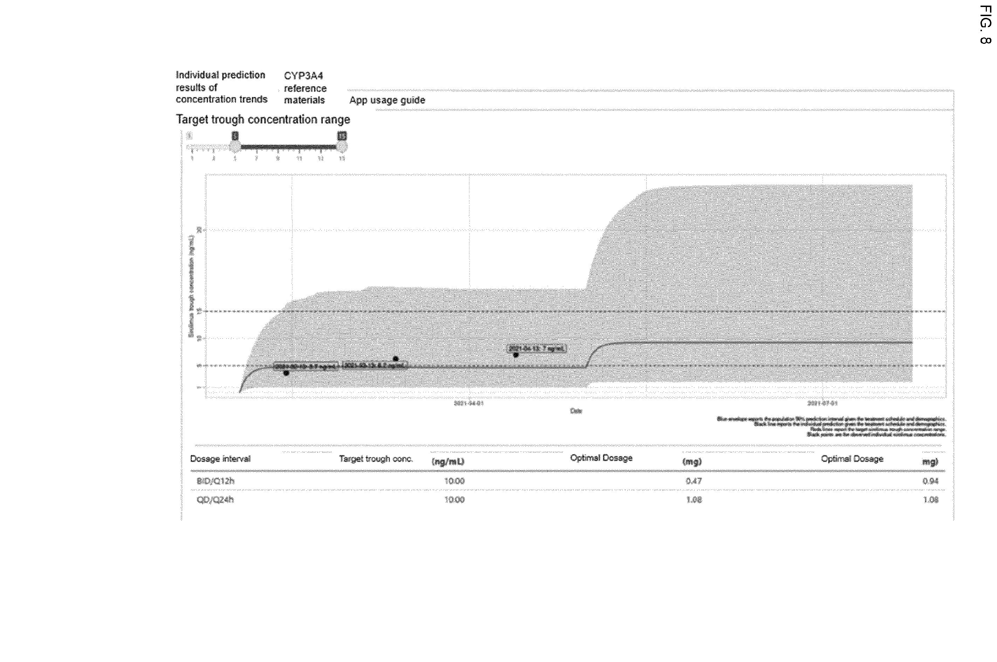

FIG. 8 is a diagram showing another example of the screen when the tab "Individual prediction results of concentration transition" is selected. In the example screen shown in FIG. 8, the transitions of the absorption phase and distribution phase of the concentration curve are no longer displayed, and only the transition of the trough concentration is displayed, and the 90% prediction interval of the blood sirolimus concentration for the same patient background is displayed as in FIG. 7. Since the trough concentration value is important for designing the dosage of sirolimus, it is convenient to be able to check the transition of the trough concentration value.

The user can select which FIG. 6 to FIG. 8 is used to display the "Individual prediction results of concentration transition". For example, an interface for switching on/off "Display concentration transition of trough concentration only" and an interface for switching on/off "Patient background" are prepared on the screen for inputting patient data. When both "Display concentration transition of trough concentration only" and "Patient background" are turned off, the screen shown in FIG. 6 is displayed. When "Display concentration transition of trough concentration only" is turned on, the screen shown in FIG. 8 is displayed. When "Patient Background" is turned on, the 90% prediction interval of the blood sirolimus concentration for the same patient background is displayed as shown in FIGs. 7 and 8.

FIG. 9 shows an example of the screen displayed when the "CYP3A4 Reference Material" tab is selected on the screens shown in FIGs. 6 to 8. The "CYP3A4 Reference Material" screen shows a table showing the degree of effect of concomitant CYP3A4 inhibitors or CYP3A4 inducers on sirolimus concentration when the patient is taking these drugs simultaneously with sirolimus administration. The table shows the rate of change of pharmacokinetic parameters when this drug is used in combination with a CYP3A4 inhibitor or CYP3A4 inducer. The rate of change is the ratio of geometric least squares (GLS) means, specifically, (this drug + concomitant drug) / (this drug alone). If there is a concomitant drug listed in the "CYP3A4 Reference Material," enter the rate of change of "CL/F" (clearance) as concomitant drug information. As a result, the value of the clearance Cl/F calculated by formula (3) is appropriately corrected based on the information of the concomitant drug. For example, when a CYP3A4 inhibitor is concomitantly used, the rate of change of CL/F is less than 1, and the inhibition of drug metabolism is reflected in the standard/patient PK parameters.

The "Guide to using the app" tab will be explained. The "Guide to using the app" tab displays the user manual of the blood sirolimus concentration estimation system.

The blood sirolimus concentration estimation system and blood sirolimus concentration estimation method have been explained above. The blood sirolimus concentration estimation system according to this embodiment calculates the age at the time of estimation, predicts the weight at the time of estimation, and calculates the standard PK parameters using the patient's age and weight at the time of estimation, and can appropriately estimate the starting dose as follows.

In another aspect of the present invention, the initial dose is determined by using patient information including at least age in months and body weight, and administration information including at least the dosage and administration timing of the drug, calculating parameters corresponding to the patient information using a blood concentration calculation formula expressing the blood sirolimus concentration based on a two-compartment model during repeated administration and a parameter calculation formula for calculating parameters to be applied to the blood concentration calculation formula, applying the parameters and the administration information to the blood concentration calculation formula to obtain a curve expressing the change in blood sirolimus concentration, and applying a target value of blood sirolimus concentration to the curve expressing the change in blood sirolimus concentration, wherein the patient's age in months used to determine the parameters at each time point is the patient's age in months at each time point calculated based on the date of birth, and the patient's weight used to determine the parameters at each time point is the patient's estimated weight at each time point estimated based on the change in the patient's past weight change.

As a preferred example, the initial dose can be the following dose. That is, (1) for patients weighing 30 kg or more, 1.4 mg/day of sirolimus. (2) for patients weighing less than 30 kg, the amount per body weight is based on the patient's age in months, with 0.02 mg/kg/day of sirolimus for patients under 3 months of age, 0.04 mg/kg/day of sirolimus for patients between 3 and 6 months of age, 0.06 mg/kg/day of sirolimus for patients between 6 and 12 months of age, and 0.08 mg/kg/day of sirolimus for patients over 12 months of age. However, the upper limit of the starting dose is 1.4 mg/day of sirolimus per patient. In other words, if the dose calculated according to the above exceeds 1.4 mg, the starting dose is 1.4 mg.

Sirolimus is thought to suppress tumors by inhibiting the action of mTOR, which regulates cell division, proliferation, survival, etc. The inventors have shown that the blood trough value of sirolimus is in the range of 5 to 15 ng/mL or a value close thereto when the drug according to the present invention, which contains sirolimus as an active ingredient, is administered orally or through a tube at the starting dose specified in the present invention. Here, the blood trough value means the minimum blood drug concentration in a steady state when the drug is repeatedly administered. The therapeutic agent for refractory lymphatic and vascular diseases according to the present invention can safely and effectively treat the disease by maintaining the blood trough value of sirolimus in such a range.

In a preferred embodiment, the therapeutic agent for refractory lymphatic and vascular diseases according to the present invention may be adjusted so that the blood trough value of sirolimus in a patient is 5 ng/mL or more and 15 ng/mL or less by increasing or decreasing the dosage based on the measured value of the blood trough concentration of sirolimus. Since the therapeutic agent for refractory lymphatic and vascular diseases according to the present invention has such a configuration, it is possible to treat the disease safely and effectively even when the systemic clearance of sirolimus is significantly different.

Specifically, after administration of the drug according to the present invention, the blood sirolimus concentration is measured when the blood sirolimus concentration approaches a steady state, and the dose can be increased or decreased under the following conditions: if the blood sirolimus concentration is 5 ng/mL or more and 15 ng/mL or less, the dose is not changed, if it is less than 5 ng/mL, the dose is increased, and if it exceeds 15 ng/mL, the dose is decreased. Here, the blood sirolimus concentration can be measured between 6 and 14 days after the start of administration at the same dose. The range of increase or decrease in the dose may be, for example, if the blood sirolimus concentration is less than 5 ng/mL, the dose is increased by 30 to 50%, and if it exceeds 15 ng/mL, the dose is decreased by 30 to 50%. The range of increase or decrease in the dosage may be selected depending on the condition of the patient, but in a preferred embodiment, the range of increase or decrease may be 30% for patients weighing less than 30 kg (i.e., 30% increase if the blood sirolimus concentration is less than 5 ng/mL, and 30% decrease if it exceeds 15 ng/mL), and 50% for patients weighing 30 kg or more (i.e., 50% increase if the blood sirolimus concentration is less than 5 ng/mL, and 50% decrease if it exceeds 15 ng/mL). In addition, the repeated administration period until the blood sirolimus concentration used to determine the increase or decrease may be a period necessary to calculate the trough value. For example, it may be set to 1 week to 3 weeks. That is, a condition may be used in which the same dosage is repeatedly administered for 1 week, etc., and the trough value of the blood sirolimus concentration is measured at that point to determine whether the dosage should be increased or decreased.

In another preferred embodiment, the therapeutic agent for refractory lymphatic and vascular diseases according to the present invention may be increased or decreased in dose based on clinical symptoms observed after repeated administration for a certain period of time. For example, if the expansion of the lesion is not suppressed after repeated administration for a certain period of time, the dosage may be increased by 30 to 50%, if the lesion has shrunk, the administration may be continued at the same dosage, and if serious side effects are confirmed, the dosage may be reduced by 30 to 50%. The range of increase or decrease in dosage may be selected according to the condition of the patient, but in a preferred embodiment, the range of increase or decrease may be 30% for patients weighing less than 30 kg (i.e., 30% increase if the expansion of the lesion is not suppressed, and 30% decrease if serious side effects are confirmed), and 50% for patients weighing 30 kg or more (i.e., 50% increase if the expansion of the lesion is not suppressed, and 50% decrease if serious side effects are confirmed).

The therapeutic agent for refractory lymphatic and vascular diseases according to the present invention is provided in any dosage form that can be administered orally or through a tube. For example, granules, syrups (including dry syrups), tablets, orally disintegrating tablets can be used, and preferably granules or syrups can be used.

The therapeutic agent for refractory lymphatic and vascular diseases according to the present invention can be manufactured by a known method according to each dosage form. For example, in the case of tablets, sirolimus can be manufactured by adding a binder or lubricant as desired to sirolimus, mixing it with an excipient, and compressing it using a tablet molding machine. Alternatively, a commercially available tablet containing sirolimus as an active ingredient may be purchased and used. In addition, in the case of granules, a solution containing sirolimus can be mixed with core particles together with a binder, and then dried. More specifically, it can be manufactured by the method described in JP 2021-88507 A. The therapeutic agent for refractory lymphatic and vascular diseases of the present invention can preferably use the sirolimus-containing granules disclosed in JP 2021-88507 A.

The therapeutic agent for refractory lymphatic and vascular diseases of the present invention contains sirolimus in an amount of about 0.01% by weight to about 20% by weight of the total composition. Preferably, the therapeutic agent for refractory lymphatic and vascular diseases of the present invention contains sirolimus in an amount of about 0.05% by weight to about 10% by weight of the total composition, and more preferably, it contains sirolimus in an amount of about 0.1% by weight to about 5% by weight.

The therapeutic agent for refractory lymphatic and vascular diseases of the present invention thus obtained can be adjusted in dosage according to the initial dosage, for example, in the case of a tablet.

In one embodiment of the present invention, the refractory lymphatic and vascular diseases targeted by the present invention are selected from Kaposiform hemangioendothelioma, tufted hemangioma, lymphangioma, lymphangiomatosis, Gorham's disease, venous malformation, blue rubber bleb nevus syndrome, mixed vascular malformation, and Klippel-Trenaunay-Weber syndrome.

Therefore, the therapeutic agent for refractory lymphatic and vascular diseases according to the present invention can be used to treat a disease selected from Kaposiform hemangioendothelioma, tufted hemangioma, lymphangioma, lymphangiomatosis, Gorham's disease, venous malformation, blue rubber bleb nevus syndrome, mixed vascular malformation, and Klippel-Trenaunay-Weber syndrome.

The initial dose of the therapeutic agent for refractory lymphatic and vascular diseases according to the present invention is set according to the patient's age and body weight, and refractory lymphatic and vascular diseases can be safely and effectively treated with less frequent blood monitoring.

On the other hand, when the therapeutic agent for refractory lymphatic and vascular diseases according to the present invention is used to treat the above-mentioned various diseases, the dosage of sirolimus may be increased or decreased based on the clinical symptoms of the disease observed after repeated administration for a certain period of time.

When a concomitant drug is used in the present invention, the method of administration of sirolimus and the concomitant drug is not particularly limited, and either sequential administration or simultaneous administration may be used.

In the present invention, even when a CYP3A4 inducer or inhibitor is used as a concomitant drug, the initial dose of sirolimus can be determined by the method of estimating the blood sirolimus concentration in the present invention, taking into consideration the use of the concomitant drug.

Furthermore, the therapeutic agent for refractory lymphatic and vascular diseases according to the present invention may be combined with surgery, nutritional therapy, radiation therapy, or the like known to those skilled in the art.

In addition, when the therapeutic agent for refractory lymphatic and vascular diseases according to the present invention is used for treatment together with the above-mentioned concomitant drug or combined with nutritional therapy, radiation therapy, or the like, the dosage of sirolimus may be increased or decreased based on clinical symptoms observed after repeated administration for a certain period of time.

[Examples]

Hereinafter, the present invention is described with reference to specific embodiments, but the present invention is not limited to the embodiments, and it is understood that various changes and modifications thereof may be made by those skilled in the art without departing from the scope or intent of the invention as set forth in the appended claims.

(Production of Sirolimus Granules)

Tocopherol (DL-ALPHA-Tocopherol, manufactured by BASF Japan Ltd.) 1.0 mg was added to 100 g of ethanol solution of sirolimus (1% by mass) and dissolved. This liquid was added to 298.0 g of mannitol while stirring in a mixer. This was transferred to a fluidized bed dryer and dried at an inlet air temperature of 70°C until the loss on drying was 0.4% or less. The resulting solid was sieved through a sieve with 1 mm openings to obtain Sirolimus Granules.

Example 1 (Administration to patients and confirmation of blood concentration)

Sirolimus granules were repeatedly administered to five patients for a certain period of time at an initial dose appropriate for their weight and age (months). Blood samples were taken 6 to 10 days after the first administration and immediately before administration on the second and subsequent days, and the sirolimus concentration was quantified by LC-MS under the following conditions to obtain blood trough level data. Administration was continued while adjusting the dosage according to the blood trough level. The results are shown in Tables 2 to 6 together with the name of the disease suffered by the patients. As shown in these tables, the therapeutic effect was confirmed in all patients by administering the therapeutic agent according to the present invention.

LC conditions

Column: ZORBAX (registered trademark) XDB-CN (inner diameter 2.1 mm, length 50 mm)
(Agilent Technologies, Inc.)
Column temperature: 50°C
Mobile phase: 0.1% acetic acid aqueous solution and 0.1% acetic acid acetonitrile solution mixed in the ratio shown in Table 1
Flow rate: 0.45 mL/min

[Table 1]

| Table 1 Gradient Program | | |
|---|---|---|
| Time (min) | 0.1% Acetic acid solution | 0.1% Acetic acid acetonitrile solution |
| 0 | 48 | 52 |
| 8 | 48 | 52 |
| 8.5 | 0 | 100 |
| 10 | 0 | 100 |
| 10.5 | 80 | 20 |

MS/MS conditions
Measurement mode: Positive ESI
Gas temperature: 350°C
Gas flow rate: 10 L/min
Nebulizer pressure: 50 psi
Capillary voltage: 4000 V

[Table 2]

| Table 2. Case 1: Klippel-Trenaunay-Weber syndrome | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 1 | 2020-08-19 | 9 months | 10.2kg | 0.61mg | 5.3 | 2020-08-26 | |
| 2 | 2020-09-02 | | | 0.61mg | 4.1 | 2020-09-02 | |
| 3 | 2020-09-16 | | | 0.79mg | 7.7 | 2020-09-16 | It is softened and the bleeding has stopped. |
| 4 | 2020-10-14 | | | 0.79mg | 9.2 | 2020-10-14 | |
| 5 | 2020-11-11 | | | 0.79mg | 5.2 | 2020-11-11 | Lesion shrinkage |
| 6 | 2020-12-09 | | | 1.03mg | 8 | 2020-12-09 | |

(continued)

| Table 2. Case 1: Klippel-Trenaunay-Weber syndrome | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 7 | 2021-01-13 | | | 1.03mg | 8.7 | 2021-01-13 | |
| 8 | 2021-02-09 | | | 1.03mg | 7.1 | 2021-02-09 | Lesion shrinkage |

[Table 3]

| Table 3 Case 2: Klippel-Trenaunay-Weber syndrome | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 1 | 2020-09-23 | 4 years old | 12.5kg | 1mg | 5.6 | 2020-10-02 | |
| 2 | 2020-10-09 | | | 1mg | 6.9 | 2020-10-09 | |
| 3 | 2020-10-22 | | | 1mg | 5.4 | 2020-10-22 | |
| 4 | 2020-11-19 | | | 1mg | 6.1 | 2020-11-19 | Improves pain and urinary retention |
| 5 | 2020-12-24 | | | 1mg | 5.3 | 2020-12-24 | |
| 6 | 2021-01-14 | | | 1.3mg | 7.2 | 2021-01-14 | |
| 7 | 2021-02-04 | | | 1.3mg | 6.7 | 2021-02-04 | |
| 8 | 2021-03-04 | | | 1.3mg | 6.3 | 2021-03-04 | |

[Table 4]

| Table 4. Case 3: Lymphangioma | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 1 | 2020-10-02 | 3 years old | 14.4 | 1.15mg | 3.2 | 2020-10-30 | |
| 2 | 2020-11-06 | | | 1.15mg | 5.1 | 2020-11-06 | |
| 3 | 2020-11-20 | | | 1.15mg | 7.8 | 2020-11-20 | |
| 4 | 2020-12-18 | | | 1.15mg | 5.8 | 2020-12-18 | Shrinking on the image |
| 5 | 2021-01-14 | | | 1.15mg | 5.2 | 2021-01-14 | |
| 6 | 2021-02-12 | | | 1.15mg | 6.4 | 2021-02-12 | |

[Table 5]

| Table 5. Case 4: Lymphangioma | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 1 | 2021-01-04 | 6 years old | 19.7 | 1.4mg | 11 | 2021-01-13 | |

(continued)

| Table 5. Case 4: Lymphangioma | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 2 | 2021-01-20 | | | 1.4mg | 9.1 | 2021-01-20 | |
| 3 | 2021-02-01 | | | 1.4mg | 8.8 | 2021-02-01 | |
| 4 | 2021-02-26 | | | 1.4mg | 8.9 | 2021-02-26 | FDP normalization, D-dimer normalization, fibrinogen increase |

Time series No. 4, the therapeutic effect "FDP normalization, D-dimer normalization, fibrinogen increase" indicates that blood coagulation abnormalities (FDP increase, D-dimer increase, fibrinogen decrease) in lymphangiomatosis were improved.

[Table 6]

| Table 6. Case 5: Vascular tumor with reduced Kasabach-merit | | | | | | | |
|---|---|---|---|---|---|---|---|
| NO. (Chronological) | Start date of administration | Months/Age (Start time) | Weight (kg) | Dosage | PK (Trough value) | PK measurement date | Treatment effect |
| 1 | 2020-12-11 | 1 year old | 8.4kg | 0.67mg | 8.8 | 2020-12-18 | Kasabach-Merritt phenomenon, improvement of coagulation abnormalities, pain relief |
| 2 | 2020-12-25 | | | 0.67mg | 7.6 | 2020-12-25 | |
| 3 | 2021-01-08 | | | 0.67mg | 7.6 | 2021-01-08 | |
| 4 | 2021-02-05 | | | 0.67mg | 5.6 | 2021-02-05 | |
| 5 | 2021-03-05 | | | 0.87mg | 6.5 | 2021-03-05 | Lesion shrinkage |

Example 2 (Confirmation by simulation using population pharmacokinetic analysis)

Population pharmacokinetic (PPK) analysis was performed using a nonlinear mixed effects model based on the whole blood sirolimus trough concentration in patients with intractable vascular and lymphatic diseases, lymphangioleiomyomatosis, etc., who were orally administered sirolimus, and healthy adults (number of cases: 215, male:female = 77:138, average age: 21.3 years), and the following variables that affect the pharmacokinetics of sirolimus were identified: body weight, age in months, blood hemoglobin concentration, dosage form of sirolimus preparation (tablet or granule), storage state of samples for blood concentration determination during transportation (refrigerated or frozen), and the presence or absence of concomitant drugs (especially CYP3A4 inducers or inhibitors). According to the above method for estimating the blood sirolimus concentration , the exposure to sirolimus was described by a two-compartment model with a first-order absorption process incorporating body weight correction and maturity correction based on the allometric law.

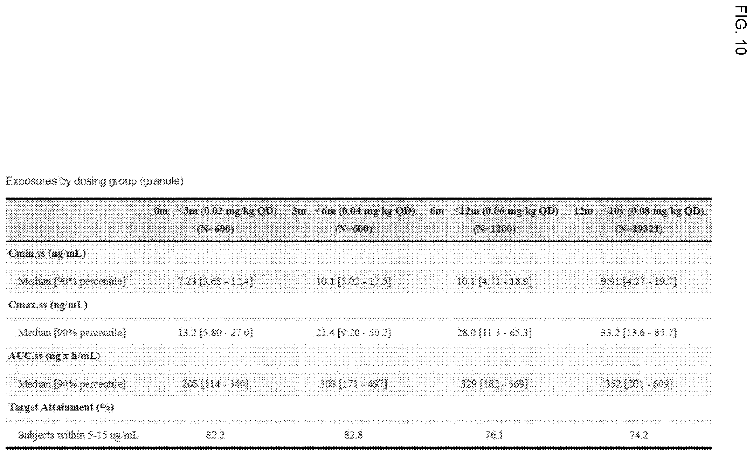

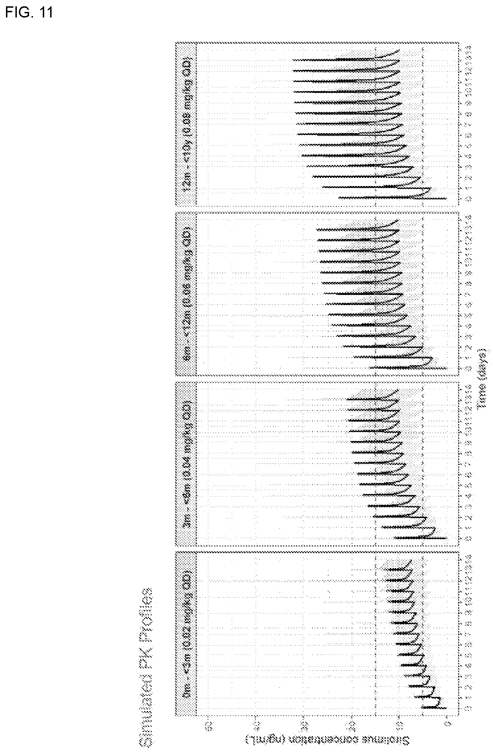

Using this model, virtual patients of age 0 to <3 months, 3 to <6 months, 6 to <12 months, and 12 months to <10 years were generated, and pharmacokinetic simulations were performed when sirolimus granules 0.2% formulation was administered once daily at 0.02 mg, 0.04 mg, 0.06 mg, and 0.08 mg (maximum 1.4 mg/person) per body weight. The results are shown in FIGs. 10 and 11. In FIG. 11, the dotted lines in each graph indicate the target sirolimus trough concentration (5 to 15 ng/mL). The solid lines indicate the average whole blood sirolimus concentration, and the base indicates the average sirolimus trough concentration. The gray area in FIG. 11 indicates the 90th percentile. As is clear from this FIG., it was confirmed that the mean whole blood sirolimus trough concentration was within the range of the target whole blood sirolimus trough concentration (5-15 ng/mL) at steady state for each of the hypothetical patients "0 to <3 months old", "3 to <6 months old", "6 to <12 months old", and "12 months to <10 years old". Furthermore, as a result of this simulation, it was estimated that the target whole blood sirolimus trough concentration (5-15 ng/mL) at steady state was maintained in 82.2%, 82.8%, 76.1%, and 74.2% of the hypothetical patients "0 to <3 months old", "3 to <6 months old", "6 to <12 months old", and "12 months to <10 years old", respectively (FIG. 10).

[Industrial Applicability]

According to the present invention, it is possible to administer an effective and minimum dose of sirolimus for treating refractory lymphatic and vascular diseases or improving symptoms, and as a result, it is possible to treat refractory lymphatic and vascular diseases or improve symptoms safely while reducing the burden on patients (particularly children including infants) and achieving their QOL.

[Brief description of the drawing]

[FIG. 1] A diagram showing the configuration of a blood sirolimus concentration estimation system.
[FIG. 2] A diagram showing a standard blood sirolimus concentration trend. The solid line is the desired trend (maximum posterior probability MAP), and the shading indicates its 90% prediction interval. The line parallel to the x-axis indicates the therapeutic range of trough values.
[FIG. 3] A diagram showing a flowchart showing the operation of a blood sirolimus concentration estimation device.
[FIG. 4] A flowchart showing the patient parameter determination process.
[FIG. 5] (a) A diagram showing an example of the change in blood sirolimus concentration calculated based on patient information. (b) A diagram showing an example of the change in blood sirolimus concentration calculated based on patient information, which is post-estimated by the Bayesian estimation method using the actual measured values of the patient. The change is specific to the patient. (c) A diagram showing the change in blood sirolimus concentration when a drug of a recommended dose corresponding to the target value of blood sirolimus concentration is administered.
[FIG. 6] A diagram showing an example of the output screen of the blood sirolimus concentration estimation system. A diagram showing the change in MAP only.
[FIG. 7] A diagram showing an example of the output screen of the blood sirolimus concentration estimation system. A diagram showing the MAP and 90% prediction interval.
[FIG. 8] A diagram showing an example of the output screen of the blood sirolimus concentration estimation system. A diagram showing the MAP and 90% prediction interval of trough values only.
[FIG. 9] A diagram showing an example of the "CYP3A4 Reference" screen. This shows the rate of change in pharmacokinetic parameters when used concomitantly with a CYP3A4 inhibitor. Of these, the information used in this app is the numerical information for CL/F.
[FIG. 10] This is a summary of parameters from a simulation of whole blood sirolimus concentration using PPK analysis.
[FIG. 11] This shows a simulation of whole blood sirolimus concentration using PPK analysis.

0m-<3m: 0 to <3 months of age, 3m-<6m: 3 to <6 months of age, 6m-<12m: 6 to <12 months of age, 12m-<10y: 12 months to <10 years of age. The horizontal dashed line indicates the target sirolimus trough concentration. The solid line is the mean whole blood sirolimus concentration, and its base indicates the mean trough concentration. The grey area indicates the 90th percentile.

[Explanation of symbols]

1 Blood sirolimus concentration estimation system
10 Input unit
11 Calculation unit
12 Memory unit
13 Output unit
20 Parameter calculation unit

21 Parameter correction unit
22 Estimated curve calculation unit
23 Recommended dose calculation unit
24 Report creation unit

**Claims**

1. A therapeutic agent, comprising sirolimus as an active ingredient, for the treatment of refractory lymphatic and vascular diseases administered by oral or tube administration with an initial dose of the dosage determined by parameters related to the pharmacokinetics of sirolimus, and the patient's body weight and age in months.

2. The therapeutic agent according to claim 1, wherein the initial dose is calculable by:

   using patient information including at least age in months and body weight, and dosing information including at least the dosage and timing of administration of the drug,
   calculating parameters corresponding to patient information using a parameter calculation formula for calculating parameters to be applied to a blood concentration calculation formula representing the blood sirolimus concentration based on a two-compartment model upon repeated administration, and
   applying the parameters and the administration information to the blood concentration calculation formula to obtain a curve showing the change in blood sirolimus concentration, and applying a target value of the blood sirolimus concentration to the curve showing the change in blood sirolimus concentration,
   wherein the patient's age in months used to determine the parameters at each time point is the patient's age at each time point calculated based on the date of birth, and the patient's weight used to determine the parameters at each time point is the patient's estimated weight at each time point estimated based on the patient's past weight change trends.

3. A therapeutic agent, comprising sirolimus as an active ingredient, for the treatment of refractory lymphatic and vascular diseases administered by oral or tube administration with an initial dose shown below depending on the weight and age in months of the patient with the disease or condition.

   (1) Patients weighing 30 kg or more: 1.4 mg/day of sirolimus
   (2) Patients weighing less than 30 kg:

      Less than 3 months old 0.02 mg/kg/day as sirolimus
      3 months to less than 6 months of age 0.04 mg/kg/day of sirolimus
      6 months to less than 12 months of age 0.06 mg/kg/day of sirolimus
      12 months and older 0.08 mg/kg/day of sirolimus
      The maximum initial dose should be 1.4 mg/day of sirolimus per patient

4. The therapeutic agent according to any one of claims 1 to 3, wherein the dosage is adjusted based on the measured blood sirolimus concentration after repeated administration of the same dosage for a certain period of time, so that the blood trough level of sirolimus in the patient is adjusted to be 5 ng/mL or more and 15 ng/mL or less.

5. The therapeutic agent according to any one of claims 1 to 3, wherein the dosage is increased or decreased based on clinical symptoms after repeated administration at the same dosage for a certain period of time.

6. The therapeutic agent according to any one of claims 1 to 5, wherein the refractory lymphatic and vascular diseases is selected from the group consisting of Kaposi type hemangioendothelioma, tufted hemangioma, lymphangioma, lymphangiomatosis, Gorham disease, venous malformation, blue rubber ball-like nevus syndrome, mixed type vascular malformation, and Klippel-Trenaunay-Weber syndrome.

FIG. 1

Patient info, administration info, dosage form info, concomitant drug info, assay info, actual values, target values

Blood sirolimus concentration estimation system

**1**

**10** Input unit

**12** Memory unit

**11** Calculation unit

**20** Parameter calculation unit

**22** Estimated curve calculation unit

**21** Parameter correction unit

**23** Recommended dose calculation unit

**24** Report creation unit

Estimated simulations, recommended doses, reports

**13** Output unit

FIG. 2

FIG. 3

```
                    ┌─────────────────────┐
                    │        START        │
                    └─────────────────────┘
                              │
                              ▼
          ┌──────────────────────────────────────────┐
          │ Patient info, administration info,        │ ─── S10
          │ dosage form info, concomitant drug info,  │
          │ assay info input                          │
          └──────────────────────────────────────────┘
                              │
                              ▼
          ┌──────────────────────────────────────────┐
          │            Target value input            │ ─── S11
          └──────────────────────────────────────────┘
                              │
                              ▼
          ┌──────────────────────────────────────────┐
          │      Standard PK parameter calculation   │ ─── S12
          └──────────────────────────────────────────┘
                              │
                              ▼
   NO            ◇ Actual measured value input? ◇      ─── S13
   ◄─────────────                                
                              │ YES
                              ▼
          ┌──────────────────────────────────────────┐
          │     Patient PK parameter calculation     │ ─── S14
          └──────────────────────────────────────────┘
                              │
                              ▼
          ┌──────────────────────────────────────────┐
          │           PK simulation drawing          │ ─── S15
          └──────────────────────────────────────────┘
                              │
                              ▼
          ┌──────────────────────────────────────────┐
          │        Recommended dose calculation      │ ─── S16
          └──────────────────────────────────────────┘
                              │
                              ▼
          ┌──────────────────────────────────────────┐
          │              Report creation             │ ─── S17
          └──────────────────────────────────────────┘
                              │
                              ▼
          ┌──────────────────────────────────────────┐
          │                  Output                  │ ─── S18
          └──────────────────────────────────────────┘
                              │
                              ▼
                    ┌─────────────────────┐
                    │         END         │
                    └─────────────────────┘
```

21

FIG. 4

Standard PK parameter calculation process

START

Calculate age at time of estimated date from date of birth — S20

Predict your future weight based on the past weight — S21

Determine standard PK parameters by applying the patient parameter calculation formula — S22

END

FIG. 5

(a)

Standard PK parameters

(b)

Patient PK parameters

(c)

Recommended doses for target values

Dose Change

FIG. 6

FIG. 7

Individual prediction results of concentration trends | CYP3A4 reference materials | App usage guide

Target trough concentration range

| Dosage interval | Target trough conc. | (ng/mL) | Optimal Dosage | (mg) | Optimal Dosage | (mg) |
|---|---|---|---|---|---|---|
| BID/Q12h | | 10.00 | | 0.47 | | 0.94 |
| QD/Q24h | | 10.00 | | 1.08 | | 1.08 |

FIG. 8

FIG. 9

Individual prediction
results of
concentration trends

CYP3A4
reference
materials          App usage guide

In the CYP3A4 column of the patient basic information, enter the clearance (CL/F) change rate when using a CYP3A4 inhibitor/inducer in combination. The default setting is 1 when not used in combination. When using a CYP3A4 inhibitor/inducer in combination, refer to the table below, check the CL/F change rate of the corresponding drug (right end of the table), and enter the value in half-width characters in the CYP3A4 column.

Table 2.7.2.3-4 Changes in the pharmacokinetic parameters of the drug in whole blood (ratio of GLS mean) when the drug is administered in combination with a drug that may interact with the drug in healthy adults

| Concomitant drug | Rate of change of pharmacokinetic parameters [1] | | | | |
| --- | --- | --- | --- | --- | --- |
| | $t_{max}$ | $C_{max}$ | $t_{1/2}$ | AUC | CL/F |
| Liquid preparation of this drug | | | | | |
| Acyclovir <See 5.4.2-2> | 0.95 | ↔ | ↔ | | ↔ |
| Atorvastatin <See 5.4.2-3> | - | - | ↔ | ↔ | ↔ |
| CsA-microemulsion formulation (coadministration) [9] | 1.92 | 2.16 | ↔ | 3.30 | 0.30 |
| CsA-microemulsion formulation (administer this drug 4 hours after CsA) [10] | 1.58 | 1.37 | 1.10 | 1.80 | 0.56 |
| CsA-microemulsion formulation (coadministration) [9] | 1.47 | 2.17 | ↔ | 2.83 | 0.35 |
| CsA-microemulsion formulation (administer this drug 2 hours after CsA) [10] | 1.47 | 2.26 | ↔ | 2.41 | 0.42 |
| CsA-microemulsion formulation (administer this drug 2 hours before CsA) [10] | 0.95 | ↔ | ↔ | ↔ | ↔ |
| Digoxin <See 5.4.2-4> | 1.03 | ↔ | ↔ | ↔ | ↔ |
| Diltiazem (2.7.2.2.2.3.1) | 1.29 | 1.43 | 0.85 | 1.60 | 0.38 |
| Erythromycin (2.7.2.2.2.3.3) | 1.40 | 4.43 | - | 4.24 | 0.24 |
| Glibenclamide <5.4.2-5 See > | ↔ | ↔ | ↔ | ↔ | ↔ |
| Ketoconazole (2.7.2.2.2.3.4) | 1.38 | 4.42 | ↔ | 10.9 | 0.085 |
| Nifedipine <See 5.4.2-6> | ↔ | ↔ | ↔ | ↔ | ↔ |
| Norgestrel/Ethinylestradiol <5.4.2-6> | - | - | 0.86 | 1.08 | ↔ |
| Rifampicin (2.7.2.2.2.3.5) | ↔ | 0.29 | ↔ | 0.18 | 5.53 |
| S-(-) Verapamil (2.7.2.2.2.3.2) | 1.08 | 2.34 | - | 2.16 | 0.46 |
| This drug oval tablet | | | | | |
| CsA-microemulsion formulation (coadministration) (2.7.2.2.2.3.6) | 0.70 | 6.12 | ↔ | 2.48 | 0.40 |
| CsA-microemulsion formulation (administer this drug 4 hours after CsA) (2.7.2.2.2.3.6) | 0.67 | 1.33 | ↔ | 1.33 | 0.75 |
| Tacrolimus (coadministration) <See 5.4.2-8> | 1.08 | ↔ | ↔ | ↔ | ↔ |
| Tacrolimus(administer this drug 4 hours after Tacrolimus) <See 5.4.2-8> | 1.28 | 1.20 | ↔ | ↔ | ↔ |

[1]: Change rate = geometric least squares (GLS) mean ratio: (this drug + concomitant drug)/(this drug alone)
↔: Indicates that the change is not statistically significant
-    : No data

FIG. 10

Exposures by dosing group (granule)

| | 0m -<3m (0.02 mg/kg QD) (N=600) | 3m -<6m (0.04 mg/kg QD) (N=600) | 6m -<12m (0.06 mg/kg QD) (N=1200) | 12m -<18y (0.08 mg/kg QD) (N=19321) |
|---|---|---|---|---|
| **Cmin,ss (ng/mL)** | | | | |
| Median [90% percentile] | 7.23 [3.60 - 12.4] | 10.1 [5.02 - 17.5] | 10.1 [4.72 - 18.9] | 9.91 [4.27 - 19.7] |
| **Cmax,ss (ng/mL)** | | | | |
| Median [90% percentile] | 13.3 [5.80 - 27.0] | 21.4 [9.70 - 50.2] | 28.9 [11.3 - 65.3] | 33.2 [13.6 - 83.7] |
| **AUC,ss (mg x h/mL)** | | | | |
| Median [90% percentile] | 208 [114 - 340] | 303 [171 - 467] | 329 [182 - 569] | 323 [201 - 609] |
| **Target Attainment (%)** | | | | |
| Subjects within 5–15 ng/mL | 52.2 | 52.8 | 76.1 | 74.2 |

FIG. 11

Simulated PK Profiles

## INTERNATIONAL SEARCH REPORT

| International application No. |
| --- |
| **PCT/JP2023/037150** |

| | |
| --- | --- |
| **A.   CLASSIFICATION OF SUBJECT MATTER** | |
| *A61K 31/436*(2006.01)i; *A61P 9/00*(2006.01)i; *A61P 9/14*(2006.01)i; *G16H 50/00*(2018.01)n<br>FI:   A61K31/436; A61P9/00; A61P9/14; G16H50/00 | |
| According to International Patent Classification (IPC) or to both national classification and IPC | |

| |
| --- |
| **B.   FIELDS SEARCHED** |
| Minimum documentation searched (classification system followed by classification symbols)<br>    A61K31/436; A61P9/00; A61P9/14; G16H50/00 |
| Documentation searched other than minimum documentation to the extent that such documents are included in the fields searched<br>    Published examined utility model applications of Japan 1922-1996<br>    Published unexamined utility model applications of Japan 1971-2023<br>    Registered utility model specifications of Japan 1996-2023<br>    Published registered utility model applications of Japan 1994-2023 |
| Electronic data base consulted during the international search (name of data base and, where practicable, search terms used)<br>    JSTPlus/JMEDPlus/JST7580 (JDreamIII); CAplus/MEDLINE/EMBASE/BIOSIS (STN) |

| | | |
| --- | --- | --- |
| **C.   DOCUMENTS CONSIDERED TO BE RELEVANT** | | |
| Category* | Citation of document, with indication, where appropriate, of the relevant passages | Relevant to claim No. |
| Y | MIZUNO, T. et al. Model-based precision dosing of sirolimus in pediatric patients with vascular anomalies. European Journal of Pharmaceutical Sciences. 2017, vol. 109, S124-S131<br>    In particular, column 3.3. | 1-6 |
| Y | HARBERS, V. E. M. et al. Effective low-dose sirolimus regimen for kaposiform haemangioendothelioma with Kasabach-Merritt phenomenon in young infants. British Journal of Clinical Pharamacology. June 2022, vol. 88, pp. 2769-2781<br>    in particular, p. 2770 | 1-6 |
| Y | 辻泰弘, 小児の成熟度を考慮した薬物動態と抗菌化学療法, 環境感染誌, 2021, vol. 36, no. 2, pp. 77-82, (TSUJI, Yasuhiro. Pharmacokinetics of Antimicrobial Chemotherapy Based on Maturation in Children. Japanese Journal of Infection Prevention and Control.)<br>    in particular, p. 79 | 1-6 |
| Y | 小関道夫, 難治性リンパ管疾患に対するシロリムス療法, 小児耳鼻咽喉科, July 2022, vol. 43, no. 1, pp. 29-34<br>    in particular, pp. 31-32, (OZEKI, Michio. Sirolimus for intractable lymphatic anomalies. Pediatric Otorhinolaryngology Japan.) | 4-6 |

☑ Further documents are listed in the continuation of Box C.     ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **25 December 2023** | **09 January 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| | International application No. |
|---|---|
| | **PCT/JP2023/037150** |

**C.    DOCUMENTS CONSIDERED TO BE RELEVANT**

| Category* | Citation of document, with indication, where appropriate, of the relevant passages | Relevant to claim No. |
|---|---|---|
| P, X | JP 2023-098709 A (NOBELPHARMA CO LTD) 10 July 2023 (2023-07-10) <br> claim 1 | 1, 2 |
| P, X | JP 2022-164134 A (TOKAI NATIONAL HIGHER EDUCATION & RES SYSTEM) 27 October 2022 (2022-10-27) <br> claims 1-4 | 3-6 |
| A | CHEN, X. et al. Initial sirolimus dosage recommendations for pediatric patients with PIK3CD mutation-related immunodeficiency disease. Frontiers in Pharmacology. September 2022, 13, 919487, doi:10.3389/fphar.2022.919487 <br> entire text | 1-6 |
| A | JP 2021-120369 A (NAT CENTER FOR CHILD HEALTH & DEVELOPMENT) 19 August 2021 (2021-08-19) <br> entire text | 1-6 |

Form PCT/ISA/210 (second sheet) (January 2015)

## INTERNATIONAL SEARCH REPORT
### Information on patent family members

International application No.

**PCT/JP2023/037150**

| Patent document cited in search report | | | Publication date (day/month/year) | Patent family member(s) | Publication date (day/month/year) |
|---|---|---|---|---|---|
| JP | 2023-098709 | A | 10 July 2023 | (Family: none) | |
| JP | 2022-164134 | A | 27 October 2022 | (Family: none) | |
| JP | 2021-120369 | A | 19 August 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021088507 A

### Non-patent literature cited in the description

- **HUBER S** ; **BRUNS CJ** ; **SCHMID G et al.** Inhibition of the mammalian target of rapamycin impedes lymphangiogenesis. *Kidney Int*, 2007, vol. 71, 771-777
- **KOBAYASHI S** ; **KISHIMOTO T** ; **KAMATA S et al.** Rapamycin, a specific inhibitor of the mammalian target of rapamycin, suppresses lymphangiogenesis and lymphatic metastasis.. *Cancer Sci.*, May 2007, vol. 98 (5), 726-33
- **BOSCOLO E** ; **COMA S** ; **LUKS VL et al.** AKT hyperphosphorylation associated with PI3K mutations in lymphatic endothelial cells from a patient with lymphatic malformation.. *Angiogenesis.*, April 2015, vol. 18 (2), 151-62
- **BOSCOLO E** ; **LIMAYE N** ; **HUANG L et al.** Rapamycin improves TIE2-mutated venous malformation in murine model and human subjects.. *J Clin Invest.*, 2015, vol. 125, 3491-604
- **LIMAYE N** ; **KANGAS J** ; **MENDOLA A et al.** Somatic Activating PIK3CA Mutations Cause Venous Malformation.. *Am J Hum Genet.*, 03 December 2015, vol. 97 (6), 914-21
- **ADRIENNE M. HAMMILL et al.** Sirolimus for the Treatment of Complicated Vascular Anomalies in Children. *Pediatr Blood Cancer*, 2011, vol. 57, 1018-24
- **OZEKI M** ; **NOZAWA A** ; **YASUE S** ; **ENDO S** ; **ASADA R** ; **HASHIMOTO H** ; **FUKAO T**. The impact of sirolimus therapy on lesion size, clinical symptoms, and quality of life of patients with lymphatic anomalies.. *Orphanet J Rare Dis.*, 13 June 2019, vol. 14 (1), 141
- *Guidelines for the Treatment of Hemangiomas, Vascular Malformations, and Lymphatic Malformations*, 2017